# EUROPÄISCHE PATENTSCHRIFT

(11) **EP 1 059 288 B1**
(45) Veröffentlichungstag und Bekanntmachung des Hinweises auf die Patenterteilung: **17.07.2002**
(21) Anmeldenummer: 00108027.4
(22) Anmeldetag: 20.04.2000
(51) Int. Cl.: C07C 319/02, C07C 323/58

(54) **Verfahren zur Ringspaltung von Thiazolidinderivaten**
Process for the ring cleavage of thiazolidine derivatives
Procédé de clivage du cycle des dérivés de la thiazolidine

(30) Priorität: 27.04.1999 DE 19919336
(43) Veröffentlichungstag der Anmeldung: 13.12.2000
(73) Patentinhaber: Consortium für elektrochemische Industrie GmbH, 81379 München (DE)
(72) Erfinder: Döring, Wolfgang, Dr., 80687 München (DE); Staudinger, Günther, 80337 München (DE)
(74) Vertreter: Potten, Holger

(56) Entgegenhaltungen:
- DE-A- 2 142 336
- DE-A- 3 607 167
- US-A- 3 300 508

## Beschreibung

Die Erfindung betrifft ein Verfahren zur hydrolytischen Ringspaltung von Thiazolidinderivaten.

Die säuren- oder basenkatalysierte Hydrolyse von Thiazolidinderivaten zu den entsprechenden (substituierten) 2-Aminomercaptanderivaten und (substituierten) Aldehyden oder Ketonen ist bekannt.

Thiazolidinderivate weisen, abhängig vom Substitutionsgrad an Position 2, eine stark unterschiedliche Hydrolysestabilität auf. Während 2,2-disubstituierte Thiazolidinderivate, die sich von den entsprechenden Ketonen ableiten, meistens recht hydrolyselabil sind, sind 2-monosubstituierte Thiazolidinderivate, die sich von den entsprechenden Aldehyden ableiten, relativ beständig gegen hydrolytische Ringspaltung. Auch 2,2-disubstituierte Thiazolidinderivate mit elektronenziehenden Gruppen in α-Stellung sind relativ hydrolysestabil.

Um wenig hydrolyseanfällige Thiazolidinderivate spalten zu können, entfernt man daher meist eines der Produkte aus dem Gleichgewicht. Bei Thiazolidinderivaten, die bei der Hydrolyse eine flüchtige Carbonylverbindung freisetzen, kann diese beispielsweise durch Destillation aus dem Gleichgewicht entfernt werden. In DE OS 1795297 wird z. B. der Isobutyraldehyd aus 2-Isopropyl-5,5-dimethyl-thiazolidin-4-carbonsäure (hydrochlorid) durch Wasserdampfdestillation entfernt, um DL-Penicillamin(hydrochlorid) zu gewinnen. Hierbei müssen für 1 mol Thiazolidinderivat ca. 25 l Wasser verdampft werden. Nachteilig ist hier die lange Reaktionsdauer bei hohen Temperaturen unter sauren oder alkalischen Bedingungen. Dies kann bei empfindlichen Verbindungen zu Zersetzung oder beispielsweise bei optisch aktiven Verbindungen zu Racemisierung führen.

Eine weitere bekannte Möglichkeit, wenig hydrolyseanfällige Thiazolidinderivate zu spalten, besteht darin, die freigesetzte Carbonylverbindung mit einem Carbonylreagens wie z. B. Hydroxylamin umzusetzen. In DE OS 2142336 ist die Ringspaltung von 2-Isopropyl-5,5-dimethyl-thiazolidinverbindungen durch Umsetzung mit einem Carbonylreagens beschrieben. Als Carbonylreagens sind z. B. Hydrazin, Phenylhydrazin, 2,4-Dinitrophenylhydrazin, Semicarbacid, Thiosemicarbacid und insbesondere Hydroxylamin geeignet. Nachteilig ist hier, daß die Carbonylverbindung anschließend schwierig oder gar nicht mehr regeneriert werden kann.

Alternativ kann die saure Hydrolyse auch bei Temperaturen oberhalb 100°C, d. h. im Autoklaven unter Druck durchgeführt werden. Dies ist z.B. in DE OS 3607167 beschrieben. Relativ lange Reaktionszeiten unter drastischen Bedingungen sind aber bei temperaturempfindlichen Verbindungen schädlich oder führen z.B. bei optisch aktiven Verbindungen zu Racemisierung.

Eine weitere Möglichkeit, wenig hydrolyseanfällige Thiazolidinderivate zu spalten, besteht darin, das freigesetzte Aminomercaptanderivat oxidativ unter Umsetzung zum entsprechenden Disulfid dem Gleichgewicht zu entziehen. Ist jedoch das Mercaptan die gewünschte Zielverbindung, so muß diese anschließend in einem weiteren reduktiven Reaktionsschritt wieder gewonnen werden. In EP 213785 A1 ist die Umsetzung von (4*R*)-Thiazolidin-4-carbonsäure mit Wasserstoffperoxid zu L-Cystin beschrieben, welches anschließend elektrolytisch zu L-Cystein reduziert wird.

Der Erfindung lag die Aufgabe zugrunde, ein Verfahren zur hydrolytischen Spaltung von Thiazolidinderivaten, insbesondere von Thiazolidin-4-carbonsäuren und Thiazolidin-4-carbonsäurederivaten bereitzustellen, das die im Stand der Technik vorhandenen Nachteile vermeidet und insbesondere sowohl das 2-Aminomercaptanderivat als auch die Carbonylverbindung in hoher Ausbeute und Reinheit liefert. Ferner sollte das erfindungsgemäße Verfahren großtechnisch einfach und kostengünstig durchführbar sein.

Gegenstand der Erfindung ist ein Verfahren zur hydrolytischen Ringspaltung von Thiazolidinderivaten zu 2-Aminomercaptanderivaten und Carbonylverbindungen, dadurch gekennzeichnet, daß
- eine wäßrige Lösung des Thiazolidinderivates mit einem sauren Kationenaustauscher in der H⁺-Form in Kontakt gebracht wird, wobei eine Lösung L₁ erhalten wird, welche die Carbonylverbindung enthält und
- der Kationenaustauscher mit einem geeigneten Elutionsmittel eluiert wird, wobei eine Lösung L₂ erhalten wird, welche das 2-Aminomercaptanderivat enthält.

Vorzugsweise werden als Thiazolidinderivate Verbindungen der allgemeinen Formel **1** eingesetzt: 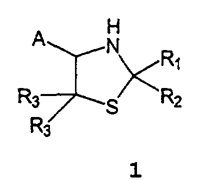
wobei A COOR₄ oder CONR₅R₆ bedeutet und
R₄ ausgewählt ist aus der Gruppe H, CN, linearer oder verzweigter C₁- bis C₁₂-Alkylrest, und
R₅ und R₆ gleich oder verschieden sind und H oder linearer oder verzweigter C₁- bis C₁₂-Alkylrest bedeuten und
R₁ und R₂ gleich oder verschieden sind und ausgewählt sind aus der Gruppe H, linearer oder verzweigter C₁- bis C₁₂-Alkylrest, ein- oder mehrfach Alkoxy- oder Hydroxy-substituierter C₁- bis C₆-Alkylrest, (CH₂)ₙCOOR₄ wobei n eine ganze Zahl von 0 bis 12 ist und R₄ die bereits genannte Bedeutung hat, Phenyl-, einoder mehrfach C₁- bis C₆-Alkyl-substituierter Phenyl-, 1-Naphthyl-, 2-Naphthyl-, 9-Phenanthrenyl-, 5- oder 6-gliedriger Heteroarylrest mit 1 bis 3 gleichen oder verschiedenen Heteroatomen aus der Gruppe O, N und S oder
die Reste R₁ und R₂ als C₂- bis C₉-Cycloalkylrest verbrückt sind und
R₃ die Bedeutung H oder Methyl hat.

Besonders bevorzugt werden Thiazolidinderivate eingesetzt, die eine Wasserlöslichkeit von mindestens 1 g/l bei 25°C und Atmosphärendruck haben.

Ganz besonders bevorzugt wird das Verfahren zur Spaltung solcher Thiazolidinderivate eingesetzt, die nach der Reaktion eine Verbindung ergeben ausgewählt aus der Gruppe Cystein, Cysteinmethylester, Cysteinethylester, Penicillamin, Penicillaminmethylester, Penicillaminethylester und das Hydrochlorid einer dieser Verbindungen.

Vorzugsweise wird der Kationenaustauscher vor dem Eluieren der Lösung L₂ mit einer Waschlösung, vorzugsweise Wasser, ausgewaschen.

Bei der erfindungsgemäßen Hydrolyse eines Thiazolidinderivats mittels eines sauren Kationenaustauschers in der H⁺-Form bindet das entstehende 2-Aminomercaptanderivat (in der protonierten Form) an den Kationenaustauscher. Es wird somit dem Gleichgewicht entzogen. Die ebenfalls entstehende Carbonylverbindung, welche nicht in kationischer Form vorliegt, bindet nicht an den Kationenaustauscher und kann leicht abgetrennt werden.

Für das erfindungsgemäße Verfahren sind alle sauren Kationenaustauscher geeignet. Saure Kationenaustauscher sind bekannt und kommerziell verfügbar. Eine Auswahl verschiedener Materialien ist in Ullmann's Encyclopedia of Industrial Chemistry, Vol. A14, S. 451 zusammengestellt. Als aktive ionenaustauschende Gruppen enthalten sie z. B. Carbonsäuregruppen (schwach saure Kationenaustauscher), Sulfonsäure- oder Phosphonsäuregruppen (stark saure Kationenaustauscher). Diese sauren Gruppen sind in der Lage, das protonierte Aminomercaptanderivat zu fixieren.

Bevorzugt werden im erfindungsgemäßen Verfahren stark saure Kationenaustauscher verwendet. Besonders bevorzugt werden stark saure Kationenaustauscher mit Sulfonsäuregruppen oder Phosphonsäuregruppen verwendet.

Die Reaktionstemperatur im erfindungsgemäßen Verfahren kann in einem weiten Bereich von vorzugsweise +5 bis +120°C variiert werden. Sie richtet sich nach der Hydrolysestabilität des eingesetzten Thiazolidinderivates. Hydrolyselabile Thiazolidinderivate wie 2,2-Dialkyl-Thiazolidinderivate werden vorzugsweise bei +5 bis +60°C, besonders bevorzugt bei +15 bis +40°C umgesetzt, während hydrolysestabile Thiazolidinderivate wie 2-Monoalkyl-Thiazolidinderivate oder mit elektronenziehenden Gruppen an 2-Position substituierte Thiazolidinderivate vorzugsweise bei +15 bis +120°C, besonders bevorzugt bei +40 bis +100°C umgesetzt werden. Die Obergrenze der Temperatur richtet sich im wesentlichen nach der dauerhaften Temperaturbeständigkeit der eingesetzten Ionenaustauscherharze, die in der Regel mit max. 120°C angegeben wird.

Der Druck kann ebenfalls in einem weiten Bereich variiert werden. Er liegt vorzugsweise in einem Bereich zwischen 0,05 und 4 bar, besonders bevorzugt bei 0,5 bis 3 bar, ganz besonders bevorzugt bei 0,8 bis 1,2 bar. Am meisten bevorzugt wird die Reaktion unter Atmosphärendruck durchgeführt.

Die verwendete Menge an Kationenaustauscher pro Mol Thiazolidinderivat richtet sich vorzugsweise nach der Kapazität des Kationenaustauschers für das Aminomercaptanderivat. Sie entspricht in der Regel etwa der Totalkapazität des Kationenaustauschers. In Abhängigkeit von der Reaktionstemperatur und der Kontaktzeit der Thiazolidinderivatlösung mit dem Austauscherharz verwendet man vorzugsweise 1 bis 10 Mol, besonders bevorzugt 1 bis 3 Mol, ganz besonders bevorzugt 1,05 bis 2 Mol Austauscherkapazität pro Mol Thiazolidinderivat.

Die Kontaktzeit der Thiazolidinderivatlösung mit dem Ionenaustauscherharz hängt ebenfalls von der Hydrolysestabilität des eingesetzten Thiazolidinderivates sowie den anderen Parametern (Temperatur, Druck, Menge Ionenaustauscher, Konzentration des Thiazolidinderivates) ab und liegt vorzugsweise zwischen 10 s und 120 min, besonders bevorzugt zwischen 1 und 60 min, ganz besonders bevorzugt zwischen 1 und 40 min.

Die Konzentration des Thiazolidinderivates richtet sich nach dessen Wasserlöslichkeit und beträgt vorzugsweise 1 bis 500 g/l, besonders bevorzugt 10 bis 300 g/l, ganz besonders bevorzugt 30 bis 200 g/l.

In einer bevorzugten Ausführungsform des Verfahrens wird der Kationenaustauscher in einer Säule mit der Thiazolidinderivatlösung durchströmt und ablaufende Fraktionen gesammelt. Anschließend wird der Kationenaustauscher mit Wasser ausgewaschen und mit einem geeigneten Elutionsmittel wieder eluiert.

Die Beladung mit der Thiazolidinderivatlösung kann aufsteigend oder absteigend erfolgen. Vorzugsweise erfolgt sie aufsteigend.

Die Elution kann im Gleichstrom oder im Gegenstrom erfolgen. Vorzugsweise erfolgt sie im Gegenstrom.

Als Elutionsmittel eignen sich wäßrige Lösungen von anorganischen Laugen wie z. B. Kalilauge, Natronlauge, Natriumcarbonat, Natriumhydrogencarbonat oder Ammoniak. Ebenso eingesetzt werden können Lösungen von Alkalimetallsalzen wie z. B. Natriumchlorid, Kaliumchlorid oder Natriumsulfat.

Bei diesem zweistufigen Verfahren muß der Kationenaustauscher nach der Elution durch Regeneration mit der wäßrigen Lösung einer anorganischen Säure wie z. B. Salzsäure oder Schwefelsäure wieder in die H⁺-Form überführt werden.

Vorzugsweise wird das Verfahren jedoch einstufig durchgeführt, d. h. die Elution des Aminomercaptanderivates erfolgt direkt mit einer Säure, vorzugsweise einer anorganischen Säure wie z. B. Salzsäure oder Schwefelsäure, wobei ablaufende Fraktionen gesammelt werden und man das Aminomercaptanderivat in Form z. B. seines Hydrochlorides oder Hydrogensulfates erhält.

Um das freie 2-Aminomercaptanderivat zu gewinnen, kann die saure Produktlösung anschließend durch Laugenzusatz oder durch Behandlung mit einem basischen Anionenaustauscher neutralisiert werden.

Die Isolierung des 2-Aminomercaptanderivates bzw. dessen Hydrochlorides oder Hydrogensulfates erfolgt mit gängigen Methoden wie z. B. Kristallisation, Extraktion oder Destillation.

Durch das einstufige Verfahren ist der Kationenaustauscher nach der Elution wieder in der H⁺-Form und steht für die nächste Umsetzung bereit.

Das erfindungsgemäße Verfahren eignet sich somit auch zur Herstellung von 2-Aminomercaptanderivaten sowie zur Herstellung der korrespondierenden Carbonylverbindungen aus Thiazolidinderivaten.

Insbesondere eignet sich das erfindungsgemäße Verfahren auch zur Gewinnung von Cystein, Cysteinmethylester oder Cysteinethylester in optisch aktiver oder racemischer Form aus einem Thiazolidinderivat der allgemeinen Formel 2 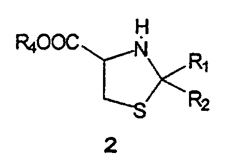 in der R1 und R2 die zu Formel 1 genannte Bedeutung haben und R4 die Bedeutung H, Methyl oder Ethyl hat.

Ferner eignet sich das erfindungsgemäße Verfahren insbesondere zur Gewinnung von Penicillamin, Penicillaminmethylester oder Penicillaminethylester in optisch aktiver oder racemischer Form aus einem Thiazolidinderivat der allgemeinen Formel 3 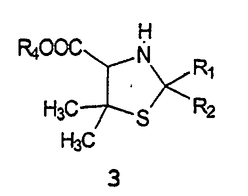 in der R1 und R2 die zu Formel 1 genannte Bedeutung haben und R4 die Bedeutung H, Methyl oder Ethyl hat.

Die folgenden Beispiele dienen der weiteren Erläuterung der Erfindung.

### Beispiel 1

### Hydrolyse von (2Ξ,4R)-2-Methyl-thiazolidin-2,4-dicarbonsäure (CP)

Unter Schutzgasatmosphäre wurde eine Lösung von 52,28 g CP in 829 g H₂O bei 90°C aufsteigend auf eine Säule gegeben, die 225 ml eines stark sauren Kationenaustauschers in der H⁺-Form (Rohm&Haas Amberjet® 1200) enthielt. Das Verhältnis Höhe :
Durchmesser der Säule betrug 4,48.
Die lineare Flußrate betrug 1,5 cm/min.
Nach beendeter Aufgabe der CP wurde im Gleichstrom mit soviel Wasser nachgefahren, daß insgesamt 2,0 kg der Lösung L₁ erhalten wurden.
Anschließend wurde ebenfalls im Gleichstrom mit 2N HCl wieder eluiert und die ablaufende Eluatlösung L₂ (1,0 l) aufgefangen.

Die Lösung L₁ enthielt laut HPLC 23,99 g Brenztraubensäure (99,6%) und weder CP noch L-Cystein.
In der Lösung L₂ waren laut HPLC 33,45 g (quant.) L-Cystein und weder Brenztraubensäure noch CP enthalten.

### Beispiel 2

### Hydrolyse von (2RS,4RS)-2-Isopropyl-5,5-dimethyl-thiazolidin-4-carbonsäure (PI)

Unter Schutzgasatmosphäre wurde eine Lösung von 55,58 g PI in 1056 g H₂O bei 90°C aufsteigend auf eine Säule gegeben, die 225 ml eines stark sauren Kationenaustauschers in der H⁺-Form (Rohm&Haas Amberjet® 1200) enthielt. Das Verhältnis Höhe :
Durchmesser der Säule betrug 4,48.
Die lineare Flußrate betrug 1,0 cm/min.
Nach beendeter Aufgabe wurde im Gleichstrom mit soviel Wasser nachgefahren, daß insgesamt 2,0 kg Spaltlösung L₁ erhalten wurden.
Anschließend wurde ebenfalls im Gleichstrom mit 2N HCl wieder eluiert und die ablaufende Eluatlösung L₂ (1,0 l) aufgefangen.

Die Lösung L₁ enthielt laut GC 19,1 g Isobutyraldehyd (96,9%). In der Lösung L₂ waren laut HPLC 40,3 g (98,8%) DL-Penicillamin und weder Isobutyraldehyd noch PI enthalten.

## Patentansprüche

1. Verfahren zur hydrolytischen Ringspaltung von Thiazolidinderivaten zu 2-Aminomercaptanderivaten und Carbonylverbindungen, **dadurch gekennzeichnet, daß**
- eine wäßrige Lösung des Thiazolidinderivates mit einem sauren Kationenaustauscher in der H⁺-Form in Kontakt gebracht wird, wobei eine Lösung L₁ erhalten wird, welche die Carbonylverbindung enthält und
- der Kationenaustauscher mit einem geeigneten Elutionsmittel eluiert wird, wobei eine Lösung L₂ erhalten wird, welche das 2-Aminomercaptanderivat enthält.

2. Verfahren nach Anspruch 1, **dadurch gekennzeichnet, daß** als Thiazolidinderivate Verbindungen der allgemeinen Formel **1** 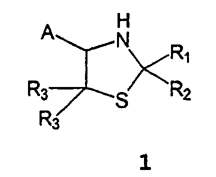
wobei A COOR₄ oder CONR₅R₆ bedeutet und
R₄ ausgewählt ist aus der Gruppe H, CN, linearer oder verzweigter C₁- bis C₁₂-Alkylrest, und
R₅ und R₆ gleich oder verschieden sind und H oder linearer oder verzweigter C₁- bis C₁₂-Alkylrest bedeuten und
R₁ und R₂ gleich oder verschieden sind und ausgewählt sind aus der Gruppe H, linearer oder verzweigter C₁- bis C₁₂-Alkylrest, ein- oder mehrfach Alkoxy- oder Hydroxy-substituierter C₁- bis C₆-Alkylrest, (CH₂)ₙCOOR₄ wobei n eine ganze Zahl von 0 bis 12 ist und R₄ die bereits genannte Bedeutung hat, Phenyl-, ein- oder mehrfach C₁- bis C₆-Alkylsubstituierter Phenyl-, 1-Naphthyl-, 2-Naphthyl-, 9-Phenanthrenyl-, 5- oder 6-gliedriger Heteroarylrest mit 1 bis 3 gleichen oder verschiedenen Heteroatomen aus der Gruppe O, N und S oder
die Reste R₁ und R₂ als C₂- bis C₉-Cycloalkylrest verbrückt sind und
R₃ die Bedeutung H oder Methyl hat.

3. Verfahren nach einem der Ansprüche 1 oder 2, **dadurch gekennzeichnet, daß** als saurer Kationenaustauscher ein stark saurer Kationenaustauscher mit Sulfonsäuregruppen oder Phosphonsäuregruppen eingesetzt wird.

4. Verfahren nach einem der Ansprüche 1 bis 3, **dadurch gekennzeichnet, daß** wobei die Reaktion bei einem Druck zwischen 0,05 und 4 bar durchgeführt wird.

5. Verfahren nach einem der Ansprüche 1 bis 4, **dadurch gekennzeichnet, daß** das Verhältnis Kationenaustauscher zu Thiazolidinderivat so gewählt wird, daß pro Mol Thiazolidinderivat 1 bis 10 Mol Austauscherkapazität vorhanden sind.

6. Verfahren nach einem der Ansprüche 1 bis 5, **dadurch gekennzeichnet, daß** die Thiazolidinlösung mit dem Kationenaustauscher eine Kontaktzeit zwischen 10 s und 120 min hat.

7. Verfahren nach einem der Ansprüche 1 bis 6, **dadurch gekennzeichnet, daß** als Elutionsmittel die wäßrige Lösung eines Alkalimetallsalzes, einer anorganischen Säure oder Lauge verwendet wird.

8. Verfahren nach Anspruche 7, wobei als Elutionsmittel wäßrige Salzsäure oder Schwefelsäure verwendet wird.

9. Verfahren zur Gewinnung von Cystein, Cysteinmethylester oder Cysteinethylester in optisch aktiver oder racemischer Form aus einem Thiazolidinderivat der allgemeinen Formel 2 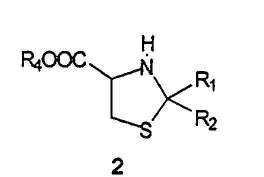 in der R1 und R2 die zu Formel 1 genannte Bedeutung haben und R4 die Bedeutung H, Methyl oder Ethyl hat, **dadurch gekennzeichnet, daß**
- eine wäßrige Lösung des Thiazolidinderivates der allgemeinen Formel 2 mit einem sauren Kationenaustauscher in H⁺-Form in Kontakt gebracht wird, wobei eine Lösung L₁ erhalten wird, welche eine Carbonylverbindung enthält und
- der Kationenaustauscher mit einem geeigneten Elutionsmittel eluiert wird, wobei eine Lösung L₂ erhalten wird, welche das Cystein enthält.

10. Verfahren zur Gewinnung von Penicillamin, Penicillaminmethylester oder Penicillaminethylester in optisch aktiver oder racemischer Form aus einem Thiazolidinderivat der allgemeinen Formel 3 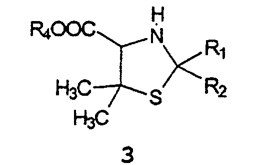 in der R1 und R2 die zu Formel 1 genannte Bedeutung haben und R4 die Bedeutung H, Methyl oder Ethyl hat, **dadurch gekennzeichnet, daß**
- eine wäßrige Lösung des Thiazolidinderivates der allgemeinen Formel 3 mit einem sauren Kationenaustauscher in H⁺-Form in Kontakt gebracht wird, wobei eine Lösung L₁ erhalten wird, welche eine Carbonylverbindung enthält und
- der Kationenaustauscher mit einem geeigneten Elutionsmittel eluiert wird, wobei eine Lösung L₂ erhalten wird, welche das Penicillamin enthält.

## Claims

1. Process for the hydrolytic ring cleavage of thiazolidine derivatives to give 2-aminomercaptan derivatives and carbonyl compounds, **characterized in that** it comprises
- bringing an aqueous solution of the thiazolidine derivative into contact with an acidic cation exchanger in the H⁺ form, giving a solution L₁ which contains the carbonyl compound and
- eluting the cation exchanger with a suitable eluent, giving a solution L₂ which contains the 2-aminomercaptan derivative.

2. Process according to Claim 1, **characterized in that** the thiazolidine derivatives used are compounds of the formula **1** 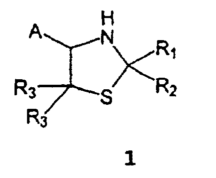
where A is COOR₄ or CONR₅R₆ and
R₄ is selected from the group consisting of H, CN, a linear or branched C₁- to C₁₂-alkyl radical, and
R₅ and R₆ are identical or different and are H or a linear or branched C₁- to C₁₂-alkyl radical and
R₁ and R₂ are identical or different and are selected from the group consisting of H, a linear or branched C₁- to C₁₂-alkyl radical, a C₁- to C₆-alkyl radical which is mono- or polysubstituted by alkoxy or hydroxyl, (CH₂)ₙCOOR₄, where n is an integer from 0 to 12 and R₄ is as defined above, a phenyl radical, and a phenyl, 1-naphthyl, 2-naphthyl, 9-phenanthrenyl radical or a 5- or 6-membered heteroaryl radical, each of which radicals is mono- or polysubstituted by C₁- to C₆-alkyl, having 1 to 3 identical or different heteroatoms from the group consisting of O, N and S or
the radicals R₁ and R₂ are bridged as a C₂- to C₉-cycloalkyl radical and
R₃ has the meaning H or methyl.

3. Process according to any of Claims 1 or 2, **characterized in that** the acidic cation exchanger used is a strongly acidic cation exchanger having sulphonic acid groups or phosphonic acid groups.

4. Process according to any of Claims 1 to 3, **characterized in that** the reaction is carried out at a pressure between 0.05 and 4 bar.

5. Process according to any of Claims 1 to 4, **characterized in that** the ratio of cation exchanger to thiazolidine derivative is chosen such that from 1 to 10 mol of exchanger capacity are present per mole of thiazolidine derivative.

6. Process according to any of Claims 1 to 5, **characterized in that** the thiazolidine solution has a contact time with the cation exchanger of between 10 s and 120 min.

7. Process according to any of Claims 1 to 6, **characterized in that** the eluent used is the aqueous solution of an alkali metal salt, an inorganic acid or base.

8. Process according to Claim 7, **characterized in that** the eluent used is aqueous hydrochloric acid or sulphuric acid.

9. Process for obtaining cysteine, cysteine methyl ester or cysteine ethyl ester in optically active or racemic form from a thiazolidine derivative of the formula 2 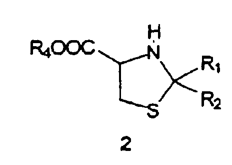 in which R1 and R2 are as defined under formula 1 and R4 has the meaning H, methyl or ethyl, **characterized in that** it comprises
- bringing an aqueous solution of the thiazolidine derivative of the formula 2 into contact with an acidic cation exchanger in H⁺ form, giving a solution L₁ which contains a carbonyl compound and
- eluting the cation exchanger with a suitable eluent, giving a solution L₂ which contains the cysteine.

10. Process for obtaining penicillamine, penicillamine methyl ester or penicillamine ethyl ester in optically active or racemic form from a thiazolidine derivative of the formula 3 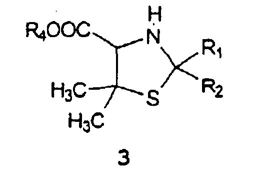 in which R1 and R2 are as defined under formula 1 and R4 has the meaning H, methyl or ethyl, **characterized in that** it comprises
- bringing an aqueous solution of the thiazolidine derivative of the formula 3 into contact with an acidic cation exchanger in H⁺ form, giving a solution L₁ which contains a carbonyl compound and
- eluting the cation exchanger with a suitable eluent, giving a solution L₂ which contains the penicillamine.

## Revendications

1. Procédé pour l'ouverture hydrolytique du cycle de dérivés de thiazolidine, conduisant à des dérivés 2-aminomercaptan et des composés carbonyle, **caractérisé en ce que**
- on met une solution aqueuse du dérivé de thiazolidine en contact avec un échangeur de cations acide sous forme H⁺, pour obtenir une solution L₁ qui contient le composé carbonyle, et
- on élue l'échangeur de cations avec un éluant approprié, pour obtenir une solution L₂ qui contient le dérivé 2-aminomercaptan.

2. Procédé selon la revendication 1, **caractérisé en ce que**, en tant que dérivés de thiazolidine, on utilise des composés de formule générale 1 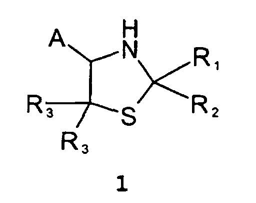
dans laquelle A représente COOR₄ ou CONR₅R₆ et
R₄ est choisi dans l'ensemble constitué par H, CN, un radical alkyle en C₁-C₁₂ linéaire ou ramifié, et
R₅ et R₆ sont identiques ou différents et représentent H ou un radical alkyle en C₁-C₁₂ linéaire ou ramifié, et
R₁ et R₂ sont identiques ou différents et sont choisis dans le groupe constitué par H, un radical alkyle en C₁-C₁₂ linéaire ou ramifié, un radical alkyle en C₁-C₆ une ou plusieurs fois substitué par un ou des groupes alcoxy ou hydroxy, (CH₂)ₙCOOR₄, n étant un nombre entier allant de 0 à 12 et R₄ ayant la signification déjà donnée, le radical phényle, un radical phényle une ou plusieurs fois substitué par un ou des groupes alkyle en C₁-C₆, le radical 1-naphtyle, 2-naphtyle, 9-phénanthrényle, un radical hétéroaryle à 5 ou 6 chaînons, comportant de 1 à 3 hétéroatomes identiques ou différents choisis dans le groupe constitué par O, N et S, ou
les radicaux R₁ et R₂ sont pontés sous forme d'un radical cycloalkyle en C₂-C₉, et
R₃ représente H ou le groupe méthyle.

3. Procédé selon la revendication 1 ou 2, **caractérisé en ce que** comme échangeur de cations acide, on utilise un échangeur de cations fortement acide comportant des groupes sulfo ou phosphonyle.

4. Procédé selon l'une quelconque des revendications 1 à 3, **caractérisé en ce qu'**on effectue la réaction sous une pression comprise entre 0,05 et 4 bars.

5. Procédé selon l'une quelconque des revendications 1 à 4, **caractérisé en ce que** le rapport de l'échangeur de cations au dérivé de thiazolidine est choisi de manière que 1 à 10 moles de capacité d'échangeur soient présentes par mole de dérivé de thiazolidine.

6. Procédé selon l'une quelconque des revendications 1 à 5, **caractérisé en ce que** la solution de thiazolidine a un temps de contact avec l'échangeur de cations compris entre 10 s et 120 min.

7. Procédé selon l'une quelconque des revendications 1 à 6, **caractérisé en ce qu'**on utilise comme éluant la solution aqueuse d'un sel de métal alcalin, d'un acide minéral ou une solution alcaline.

8. Procédé selon la revendication 7, dans lequel on utilise comme éluant une solution aqueuse d'acide chlorhydrique ou sulfurique.

9. Procédé pour l'obtention de cystéine, d'ester méthylique de cystéine ou d'ester éthylique de cystéine, sous forme optiquement active ou racémique, à partir d'un dérivé de thiazolidine de formule générale 2 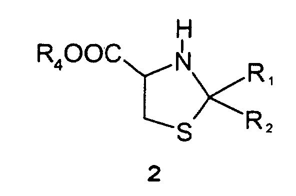 dans laquelle R₁ et R₂ ont les significations données à propos de la formule 1 et R₄ représente H, le groupe méthyle ou éthyle, **caractérisé en ce que**
- on met une solution aqueuse du dérivé de thiazolidine de formule générale 2 en contact avec un échangeur de cations acide sous forme H⁺, pour obtenir une solution L₁ qui contient un composé carbonyle, et
- on élue l'échangeur de cations avec un éluant approprié, pour obtenir une solution L₂ qui contient la cystéine.

10. Procédé pour l'obtention de pénicillamine, d'ester méthylique de pénicillamine ou d'ester éthylique de pénicillamine, sous forme optiquement active ou racémique, à partir d'un dérivé de thiazolidine de formule générale 3 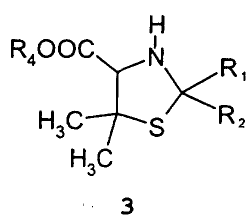 dans laquelle R₁ et R₂ ont les significations données à propos de la formule 1, et R₄ représente H, le groupe méthyle ou éthyle, **caractérisé en ce que**
- on met une solution aqueuse du dérivé de thiazolidine de formule générale 3 en contact avec un échangeur de cations acide sous forme H⁺, pour obtenir une solution L₁ qui contient un composé carbonyle, et
- on élue l'échangeur de cations avec un éluant approprié, pour obtenir une solution L₂ qui contient la pénicillamine.
